# EUROPEAN PATENT SPECIFICATION

(11) **EP 0 670 712 B1**
(45) Date of publication and mention of the grant of the patent: **28.08.1996**
(21) Application number: 94901832.9
(22) Date of filing: 18.11.1993
(51) Int. Cl.: A61K 7/48, A61K 7/06

(54) **COSMETIC COMPOSITION**
KOSMETISCHE MITTEL
COMPOSITION COSMETIQUE

(30) Priority: 24.11.1992 GB 9224563
(43) Date of publication of application: 13.09.1995
(73) Proprietor: UNILEVER PLC, London EC4P 4BQ (GB); UNILEVER N.V., 3013 AL Rotterdam (NL)
(72) Inventor: CORDERY, Caroline Susan, Altrincham, Cheshire WA15 0SL (GB); DAWSON, Peter Leonard, Upton, Chester CH2 1EA (GB)
(74) Representative: Mulder, Cornelis Willem Reinier, Dr.
(86) International application number: EP9303255
(87) International publication number: WO9412151

(56) References cited:
- EP-A- 0 104 679
- EP-A- 0 490 103
- FR-A- 2 563 104
- PATENT ABSTRACTS OF JAPAN vol. 9, no. 317 (C-319) & JP,A,60 152 407 (SHISEIDO) 10 August 1985

## Description

The present invention relates to cosmetic compositions, particularly to exfoliating compositions for use on the hair and scalp, or the body.

Conventional exfoliating cosmetic compositions are commonly known as "facial scrubs" and are used principally on the face. Such compositions may, by abrasion, remove dead or unwanted cells from the surface of the skin. Throughout this specification the term "exfoliating" includes both this abrasive action and the massaging of the skin or scalp.

The exfoliating properties of the known compositions are provided by solid abrasive particles suspended therein. Two known particulate abrasive materials used in the known epidermal skin exfoliating compositions are calcium carbonate and the endocarp of apricot seeds. In order to provide the necessary abrasive characteristics, the particles generally have a size of at least 40 microns and are solid and relatively dense, hence their use generally with some sort of suspending system.

During our experiments to investigate the applicability of these known exfoliating compositions to use on the scalp, i.e. to their use as shampoos or other hair care products, it was found that the inherent permanent grittiness of the suspended abrasive particles gave rise to an undesirable product having poor aesthetic acceptability.

On the other hand, known abrasive materials which are insoluble under saturation conditions but which dissolve on aqueous dilution, for example potassium sulphate, give such a transient abrasive effect when delivered from a shampoo composition, chat their practical exfoliating/massaging usefulness is severely limited.

In addition, Patent Abstracts of Japan vol. 9 no. 317 (C-319) & JP-A-60152407 (Shiseido) describes a skin washing composition which has both a washing and massaging effect, and provides an improved feeling of satisfaction after use by blending an external preparation with granules which have previously been granulated with a water insoluble binder. The granules may comprise 0.1-50% by weight of the composition, and have a particle size in the region 0.05-2.0 mm, the granules being such that they lessen gradually during use.

It was therefore an aim of the present inventors to devise a form of exfoliating/massaging cosmetic product directed in particular to use on the scalp, which, while having good exfoliating or massaging properties, avoids the inherent grittiness of the suspended abrasive particles of the prior art.

Surprisingly, it has now been found that the above object may be fulfilled by using as the exfoliating/massaging material abrasive particles which are friable under the conditions of use of the composition.

According to the present invention there is provided an exfoliating or massaging cosmetic composition including a particulate exfoliating or massaging material, characterised in that the particles of the material are friable under conditions of use of the composition.

When used in the form of a hair care composition, the compositions of the invention are useful not only in providing an exfoliating or massaging effect on the scalp, but also in aiding the removal from the hair of materials such as resins or other hair cosmetic substances, which have been deposited on the hair to provide styling or conditioning benefits.

The invention will now be described in detail.

Although the exfoliating/massaging compositions according to the invention are particularly attractive for use on the scalp, in which regime conventional exfoliating products are unsatisfactory as explained above, the exfoliating/massaging compositions of this invention are also useful for application to the skin, where it is also possible to exploit the effects of temporary grittiness.

In accordance with the invention, when a cosmetic composition such as a shampoo or conditioner is massaged onto the scalp, or a face or body shampoo is worked on the skin with the fingers, the shear and crush forces which are created are sufficient to cause the particles of exfoliating/massaging material to break up after a period of time, preferably a short period of time, whereby the gritty feel experienced by the user is eliminated.

Preferably, the particles of exfoliating/ massaging material are friable under shear and/or crush forces of a magnitude near or towards an upper limit of those created during a normal use regime of the composition in which the particles are incorporated. Since the forces created at any particular location during such a regime are constantly varying and may only rarely reach a maximum, this may have the effect of delaying the onset of break up of the particles, so that there is a period at the beginning of the use regime when the particles remain whole and are able to perform their exfoliating/massaging function with maximum effect. Thus, the exfoliating/massaging power of the composition may be controlled to some extent by the physical strength of the particles.

More generally however, it is likely to be sufficient for the exfoliating/massaging particles to be friable merely within the range of shear and/or crush forces normally produced in the relevant use regime, since the considerably variable forces produced at a particular location over time enable at least some of the particles to survive intact long enough to perform their exfoliating/massaging function to a satisfactory degree.

It is possible to design the breakdown time of the particles by controlling their average crush strength. This can be done by selecting a particular particulate material or by carrying out structural modification during the manufacturing process as explained further below. Loss of palpability of the particles in use may provide the further advantage of a tactile indication that sufficient massaging or working in has taken place, which will encourage good product usage and efficiency.

The strength of the particles may be measured as single particle crush strengths or by uniaxially confined compaction techniques. In the latter method, average particle strengths can be expressed as the Lüdde and Kawakita Relationship, as described in Powder Tech. (1970/71), 4, 61-68, "Some considerations on powder compression equations", K. Kawakita & K.-H. Lüdde (the disclosure of which is incorporated herein by reference).

We have found that particles exhibiting Lüdde and Kawakita strengths in the range of from about 5 to about 40 MPa (at 50% relative humidity and 294K), more preferably in the range of from about 10 to about 35 MPa, are suitable for use in the present invention. The lower limit is set by the process used to make the cosmetic composition, it being critical that the agglomerate is not destroyed to any significant extent during the processing. The upper limit is set by the crush forces that normally occur during the intended use of the composition. The upper limit should be below theses forces so that the agglomerates are satisfactorily crushed and broken down during use.

The particle size of the material is chosen to give a satisfactory exfoliating effect on the scalp or the skin. Particle sizes in the range of from about 0.03 to about 3 mm are preferred, more preferably from about 0.05 to about 2 mm, most preferably from about 0.1 to about 1 mm.

When the exfoliating particles break up under the action of shear and crush forces, the resulting average particle size (diameter) will typically be less than about 40 microns. Particles of such small sizes do not feel gritty.

The material used in the invention may be any which has sufficient hardness to perform an exfoliating function, yet has a structure which exhibits friability and enables the particles to break up under conditions of normal use of the composition in which they are incorporated.

Suitable materials include certain silicas, aluminas and other crystalline or amorphous materials having abrasive properties. Particularly preferred are certain amorphous silicas, such materials generally being inert and therefore suffering from few or no safety problems.

One preferred particulate exfoliating material suitable for use in the invention is a structurally modified silica derived from the thickening silica sold under the trade mark Sident 22S (ex Degussa). Other suitable silicas include Zeosyl 200 (ex Zeofinn), Sorbosil TC 15 (ex Crosfield Chemicals), Tixosil 333 (ex Rhone-Poulenc), Silox 15X (ex W.R. Grace).

For use in the present invention, these commercially available particulate silicas may be structurally modified by pre-agglomeration, for example by exposure to moisture and subsequent drying.

A characteristic feature of these amorphous silicas which makes them suitable for pre-agglomeration in this manner is their very low primary particle size which is typically in the range of from about 0.01 to 0.2 microns, more usually from about 0.01 to 0.1 microns and most typically in the region of about 0.05 microns, and also their high pore volumes, typically in the range of from about 1 to 3 cm³g-¹, more usually in the region of about 2 cm³g-¹ (as measured by mercury porosimetry and BET nitrogen techniques). Surface areas, measured for example by nitrogen adsorption, may preferably be in the range of from about 100 to about 300 m²g-¹, more preferably in the region of about 250 m²g⁻¹.

Owing to the porous nature of the agglomerated silica particles, it is possible for them to act as delivery vehicles for substances such as colouring pigments, or cosmetic or pharmaceutical actives such as anti-dandruff actives. One such particulate anti-dandruff active is Zinc Pyrithione. Other materials that may be incorporated in this way are: zinc citrate, anti-microbial agents and fragrances and flavours. Such substances may be introduced within the pores of the friable material by conventional techniques) and, as the particles break up during use of the composition, released therefrom onto the scalp and hair, or skin.

Agglomeration using water and subsequent drying of the modified structure thus formed produces particles of a considerably larger size, but which are friable under relatively low shear forces so as to break up into smaller constituent parts.

Suitable industrial processes for forming structurally modified particulate abrasive materials such as that described above include: spray drying from a body of the particulate material wetted with a suitable amount of water or other volatile liquid; granulation, similar to that used in the preparation of solid detergents in granular form; and pressure compaction. Such processes are well known. One preferred method, for the purpose of illustration only, is as follows:

Approximately 1000 cm³ of Sident 22S is placed in a double bladed high speed mixer. Then, with the motor running, water is added until a critical point is reached when the silica changes from a free flowing powder to agglomerated particles. The volume of water required varies, depending on the initial moisture content of the silica. The range of about 170 to about 240 cm³ of water may be typical for 1000 cm³ of Sident 22S. The agglomerated particles are then dried.

In the exfoliating cosmetic compositions according to the present invention, the level of particulate material may be wide ranging, for example depending upon the physical form of the desired end product.

Preferably the particulate material is suspended in a liquid composition. By suitable adjustment of the solid to liquid ratio, and the viscosity of the liquid phase, the composition may take a physical form anywhere between a thick paste or gel and a low viscosity liquid.

In the preferred compositions of the invention the particulate material is insoluble in the base composition. In this context, "insoluble" means having sufficiently low solubility at ambient temperature that the solid particles remain undissolved or substantially undissolved in the composition, such that their friability under the conditions of use of the composition and thus their ability to perform their exfoliating function are not adversely affected. Preferably, the particulate material should also be insoluble in the generally more aqueous environment in which the composition is used.

In preferred embodiments of the invention, the particulate material is present in the compositions in an amount of from about 1 to about 99% by weight, more preferably from about 2 to about 60%, even more preferably from about 3 to about 40%. In liquid compositions the latter range is particularly suitable.

The exfoliating cosmetic compositions of the invention may contain one or more additional components, depending on the intended end use of the product, as will now be described.

Cleaning compositions preferably also comprise one or more surfactants, preferably selected from anionic, nonionic, amphoteric and zwitterionic surfactants, and mixtures thereof. Any surfactant or mixture of surfactants can be used provided that the solubility criterion described above are fulfilled.

The surfactant(s) may be present in the cosmetic compositions of the invention in a total amount of from about 1 to about 50% by weight, preferably from about 2 to about 30% by weight.

Water is another preferred component of the compositions of the invention and may be present in an amount of from about 10 to about 90% by weight, preferably from about 20 to about 80%, more preferably from about 40 to about 75%.

In compositions of the present invention it is particularly preferred that one or more thickening agents or suspending agents are included, in order that the particulate material remains stably dispersed throughout the composition.

One preferred method of suspension is via surfactant lamellar phase formation in the composition, in which the particles of exfoliating material are prevented from settling and remain suspended in the composition.

Suitable electrolytes to achieve lamellar surfactant phases include water soluble alkali metal or ammonium salts, e.g. a chloride, sulphate or citrate.

Another preferred method of suspension is by the use of swelling clays to form ordered networks which prevent the particles of material from settling out under the force of gravity. Suitable swelling clays include for example Laponite (ex Laporte).

Other suitable suspending agents are well known in the art and include for example polyacrylic acid, copolymers and cross-linked polymers of acrylic acid, copolymers of acrylic acid with a hydrophobic monomer, copolymers of carboxylic acid-containing monomers and acrylic esters, cross-linked copolymers of acrylic acid and acrylate esters, esters of ethylene glycol or esters of polyethylene glycol (e.g. fatty acid esters thereof), heteropolysaccharide gums, and certain cellulose derivatives such as sodium carboxymethyl cellulose.

Polyacrylic acid is available commercially as Carbopol 420, Carbopol 488 or Carbopol 943 (ex Goodrich). Suitable polymers of acrylic acid cross-linked with a polyfunctional agent include those available commercially as Carbopol 910, Carbopol 934, Carbopol 940 and Carbopol 941 (ex Goodrich).

An example of a suitable copolymer of a carboxylic acid containing monomer and acrylic ester is Carbopol 1342 (ex Goodrich). Suitable examples of cross-linked polymers of acrylic acid and acrylate esters are Pemulan PR1 and Pemulan PR2. Suitable heteropolysaccharide gums include xanthan gum and guar gums.

A further class of suitable suspending agents are those materials which function as pearlescing agents in cosmetic compositions.

The pearlescing agent may be selected from a wide range of pearlescing agents, for example C₁₆-C₂₂ fatty acids, C₁₆-C₂₂ esters of fatty acids with alcohols and C₁₆-C₂₂ esters of fatty acids incorporating elements such as alkylene glycol units and the like. Suitable alkylene glycol units include ethylene glycol and propylene glycol, though higher alkylene chain length glycol may also be employed. Suitable higher alkaline chain length glycol include polyethylene glycol and polypropylene glycol and the like. Preferably, the pearlescing agent is selected from polyethylene glycol mono or di-esters of C₁₆-C₂₂ fatty acids having from 1 to 7 ethylene oxide units.

The thickening agent or suspending agent, which may be used singly or as mixtures of two or more such materials, may be present in the composition in a total amount of from about 0.1 to about 60% by weight; preferably from about 5 to about 20% for surfactant suspension; preferably from about 0.5 to about 5% for swelling clay suspension; and preferably from about 0.1 to about 5% for polymer suspension.

The compositions of the invention may contain other components conventionally found in cosmetic compositions for the hair or the skin. Suitable additional ingredients include: one or more hair conditioning agents, e.g. cationic surfactants, cationic polymers, volatile silicones, non-volatile silicones, quaternary ammonium salts having at least one long chain alkyl or alkenyl group, protein hydrolysates, quaternized protein hydrolysates; perfumes; opacifiers; colourings; preservatives; antidandruff agents; skin moisturising agents; herb and plant extracts; essential oils; proteins; pH adjusting agents; and anti-microbials.

Further optional ingredients which may be used in compositions according to the invention include one or more conventional abrasive materials such as those normally found in prior art exfoliating compositions. Such materials may for example be used in combination with the friable particulate material to give enhanced exfoliating and/or massaging benefits, for instance where a particularly heavy exfoliating/massaging effect is required.

Compositions in accordance with the present invention may be made by conventional methods of preparing cosmetic compositions, eg facial scrubs. If suspension is through surfactant lamellar phase formation, however, it is preferable that the particulate material is incorporated in the composition prior to the formation of the lamellar phase which stabilises the dispersed particles, in order for the particulate material to be successfully and stably incorporated therein. Alternatively, for creams and pastes, the base composition may be prepared by mixing the base ingredients, with addition of thickener or suspending agent if used, followed by low shear mixing in of the pre-prepared particulate material.

It is important in the preparation of compositions in accordance with the invention that any mixing is done at a sufficiently low shear that the friable particulate material does not experience forces sufficiently great to cause many of the particles to fracture.

A preferred preparative procedure for a preferred composition comprises the following steps:
(a) Dissolve salt or other lamellar phase-forming component in water, without heating;
(b) Add preservative, antioxidant, if used;
(c) Add particulate material with mixing;
(d) Add surfactant;
(e) Add any other remaining optional ingredients, eg opacifier, pearlescer, colourant, perfume, etc.;
(f) Finally add any co-surfactant (e.g. betaine).

If, however, suspension is to be achieved via a structured network formed by a swelling clay, then the clay sol must first be prepared and the surfactant and particulate material then added without gelation or aeration, to form a stable product.

Accordingly, in this context a preferred preparative procedure may comprise the following steps:
(a) Prepare sol by introducing swelling clay into pre-warmed water with vigorous stirring and allow to cool;
(b) Dilute surfactant using high shear mixer;
(c) Mix sol and water;
(d) Add surfactant;
(e) Add particulate material with mixing;
(f) Add preservatives and any other remaining optional ingredients e.g. opacifier, pearliser, colourant, perfume etc.;
(g) Add any cosurfactant;
(h) Finally add salt if necessary to correct viscosity.

The exfoliating cosmetic compositions of the invention may be used in a similar way to conventional exfoliating compositions, and when directed to use on the scalp they may be used in a similar manner to conventional shampoos or conditioners; i.e. a suitable amount of the composition is applied to wetted or washed hair and the composition massaged on the head so as to exfoliate the scalp and clean the hair. Owing to the friability of the particulate material, any grittiness experienced by the user will soon disappear, so that, once the particulate material has performed its exfoliating function, the composition is still available for further massaging or working in, as necessary, for example for delivering one or more additional benefits from other components in the composition.

The invention is further illustrated by the following non limiting Examples.

### Example 1 (Shampoo)

| Component | %wt |
|---|---|
| Sodium lauryl ether sulphate 2EO | 12.0 |
| Cocoamidopropyl betaine | 2.0 |
| Cyclomide CD⁽¹⁾ | 1.0 |
| Particulate material (100-1000µ) | 5.0 |
| Sodium chloride | 14.0 |
| Perfume, colouring, preservative | qs |
| Water | to 100 |

| | |
|---|---|
| ⁽¹⁾ Coconut diethanolamide (ex Cyclo) | |

### Example 2 (Shampoo)

| Component | %wt |
|---|---|
| Ammonium lauryl sulphate | 12.0 |
| Lauryl betaine | 4.0 |
| Particulate material (500-700µ) | 10.0 |
| Sodium chloride | 14.0 |
| Perfume, colouring, preservative | qs |
| Water | to 100 |

### Example 3 (Shampoo)

| Component | %wt |
|---|---|
| Sodium lauryl ether sulphate 3EO | 14.0 |
| Cocoamidobetaine | 2.0 |
| Particulate material (700-1000µ) | 5.0 |
| Laponite XLS | 1.5 |
| Sodium chloride⁽²⁾ | 1.0 |
| Perfume, colouring, preservative | qs |
| Water | to 100 |

| | |
|---|---|
| ⁽²⁾ Additional thickener | |

The particulate material used for examples 1 to 3 was Sident 22S which has a primary particle size of 0.02-0.04: Sieving was used to obtain the different size fractions tested. The primary particle size of other suitable 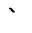 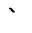 thickening' type silicas eg Tixosil 333 (Rhone-Poulenc), Sorbosil TC15 (Crosfield Chemicals) also fall in the range 0.01-0.1µ. These suitable materials are also characterised by possessing Pore Volumes >1.5 cm³g⁻¹ and Linseed Oil Absorptions >150 g/100g.

To test for friability of the agglomerated particles in use, 1.0g of the shampoo formulated according to Example 1 and containing 0.05g (5% w/w dry) of the agglomerates of particle size 500-700µ was washed into 10g hair by hand for 30 seconds. The hair was rinsed and all the rinsings from the hair sample were passed through a 250µ aperture sieve; the particles retained were dried at 50°C to constant weight. The test was carried out in triplicate. The average weight of particles >250µ following washing was 0.003g, indicating approximately 95% breakdown of agglomerates under these conditions.

A video recording was made of the scalp treated with the shampoo according to the invention compared with the other half of the scalp which was treated with a conventional non-exfoliating shampoo. The exfoliating shampoo was seen to remove more of the superficial layers of skin.

### Example 4 (Shampoo)

| Component | %w |
|---|---|
| Sodium lauryl sulphate 2EO | 12.0 |
| Cocoamidobetaine | 2.0 |
| Cyclomide CD | 1.0 |
| Particulate material (500-700µ) | 10.0 |
| Laponite XLS | 1.5 |
| Kelzan S | 0.2 |
| Sodium chloride⁽³⁾ | 1.0 |
| Perfume, colouring, preservative | qs |
| Water | to 100 |

| | |
|---|---|
| ⁽³⁾ Additional thickener | |

### Example 5 (Shower gel)

| Component | %w |
|---|---|
| Genopol ZRO⁽⁴⁾ | 11.7 |
| Cocoamidopropyl betaine | 1.8 |
| Particulate material (100-500µ) | 10.00 |
| Laponite XLS | 1.35 |
| Sodium chloride | 1.15 |
| Perfume, colouring, preservative | qs |
| Water | to 100 |

| | |
|---|---|
| ⁽⁴⁾ Sodium lauryl ether sulphate 3EO (ex Hoechst) | |

### Example 6 (Shower gel)

| Component | %w |
|---|---|
| Genopol ZRO | 11.3 |
| Cocoamidopropyl betaine | 1.8 |
| Particulate material (100-500µ) | 5.0 |
| Sorbosil TC 15⁽⁵⁾ | 8.0 |
| Sodium chloride | 13.0 |
| Opacifier⁽⁶⁾ | 1.0 |
| Perfume, colouring, preservative | qs |
| Water | to 100 |

| | |
|---|---|
| ⁽⁵⁾ thickener | |
| ⁽⁶⁾ e.g. ethylene glycol distearate | |

### Example 7 (anti-dandruff shampoo)

A test was carried out to check that anti-dandruff particles could be beneficially incorporated into the agglomerate and delivered from a shampoo. The shampoo base was the same as that used in Example 1 and the silica was the same as that used for Example 2. The ratio of Silica to anti-dandruff particles was 10:1. 90% of the anti-dandruff particle passed through a 20 mesh sieve (841 micron ASTM E-11-61). The agglomerate was used at levels to give 5 and 10% exfoliating particles in the shampoo. Human hair was washed with the shampoo. Use of an agglomerate enables targeted delivery of the anti-dandruff active to the scalp and base of the hairs. Incorporation within an inert silica granule enables relatively high levels of potentially harmful materials such as Zinc Pyrithione to be delivered from a shampoo. The exfoliant also assists in delivery of the anti-dandruff active to the hair follicle by partially removing sebum which would otherwise plug the follicle and prevent ingress of the active ingredient.

## Claims

1. An exfoliating composition including a particulate exfoliating material with a particle size in the range 0.03 to 3 mm., characterised in that the particulate material comprises an agglomerated silica having a primary particle size in the range 0.01 - 0.2 microns, which is friable and under conditions of use of the composition break up into particles having an average size of less than 40 microns.

2. A cosmetic composition according to claim 1 which is in the form of a hair care composition, wherein the particulate material is friable under conditions of use of the composition on the scalp and hair.

3. A cosmetic composition according to claims 1 or 2, wherein the particulate material has a Lüdde & Kawakita strength (as herein defined) in the range 5 to 40 MPa at 50% relative humidity and 294 K.

4. A cosmetic composition according to claim 1, wherein the particulate material is insoluble in the composition both before and during use.

5. A cosmetic composition according to claim 1, further comprising one or more surfactants selected from anionic, nonionic, amphoteric and zwitterionic surfactants, and mixtures thereof.

6. A cosmetic composition according to claim 1, further comprising one or more thickening agents or suspending agents.

7. A cosmetic method of removing dead or unwanted cells from the surface of the scalp or the skin, comprising applying thereto and massaging or working thereon an exfoliating composition including a particulate exfoliating material with a particle size in the range 0.03 to 3 mm., characterised in that the particulate material is friable and under conditions of use of the composition breaks up into particles having an average size of less than 40 microns.

8. Use as an exfoliating material in a cosmetic composition of a particulate material comprising particles which are friable under conditions of use of the composition.

## Patentansprüche

1. Exfoliationsmittel, das ein teilchenförmiges Exfoliationsmaterial mit einer Teilchengröße im Bereich 0,03 bis 3 mm einschließt, dadurch gekennzeichnet, daß das teilchenförmige Material ein agglomeriertes Siliciumdioxid mit einer Primärteilchengröße im Bereich 0,01 bis 0,2 µm umfaßt, das zerreibbar ist und unter den Anwendungsbedingungen des Mittels in Teilchen mit einer mittleren Größe von weniger als 40 µm zerfällt.

2. Kosmetisches Mittel nach Anspruch 1 in Form eines Haarpflegemittels, wobei das teilchenförmige Material unter den Anwendungsbedingungen des Mittels auf der Kopfhaut und dem Haar zerreibbar ist.

3. Kosmetisches Mittel nach Ansprüchen 1 oder 2, wobei das teilchenförmige Material eine Lüdde & Kawakita-Festigkeit (wie hier definiert) im Bereich 5 bis 40 MPa bei 50 % relativer Luftfeuchtigkeit und 294K aufweist.

4. Kosmetisches Mittel nach Anspruch 1, wobei das teilchenförmige Material in dem Mittel vor und während der Verwendung unlöslich ist.

5. Kosmetisches Mittel nach Anspruch 1, das außerdem ein oder mehrere Tenside, ausgewählt aus anionischen, nichtionischen, amphoteren und zwitterionischen Tensiden und Gemischen davon umfaßt.

6. Kosmetisches Mittel nach Anspruch 1, das außerdem ein oder mehrere Verdickungsmittel oder Suspendierungsmittel umfaßt.

7. Kosmetisches Verfahren zur Entfernung von abgestorbenen oder unerwünschten Zellen von der Oberfläche von Kopfhaut oder Haut, umfassend Auftragen und Einmassieren oder Einarbeiten eines Exfoliationsmittels, das ein teilchenförmiges Exfoliationsmaterial mit einer Teilchengroße im Bereich von 0,03 bis 3 mm einschließt, dadurch gekennzeichnet, daß das teilchenförmige Material zerreibbar ist und unter den Anwendungsbedingungen des Mittels in Teilchen mit einer mittleren Größe von weniger als 40 µm zerfällt.

8. Verwendung eines teilchenförmigen Materials, umfassend Teilchen, die unter den Anwendungsbedingungen des Mittels zerreibbar sind, als Exfoliationsmaterial in einem kosmetischen Mittel.

## Revendications

1. Composition exfoliante comportant une matière exfoliante particulaire avec une granulométrie dans la gamme des 0,03 à 3 mm, caractérisée en ce que la matière particulaire comprend une silice agglomérée ayant une granulométrie primaire dans la gamme des 0,01 à 0,2 microns, qui est friable et dans des conditions d'utilisation de la composition se rompt en particules ayant une dimension moyenne inférieure à 40 microns.

2. Composition cosmétique selon la revendication 1, qui est sous forme d'une composition de soin des cheveux, dans laquelle la matière particulaire est friable dans les conditions d'utilisation de la composition sur le cuir chevelu et les cheveux.

3. Composition cosmétique selon la revendication 1 ou 2, dans laquelle la matière particulaire a une résistance Lüdde et Kawakita (comme décrit ici) dans la gamme de 5 à 40 MPa à 50% d'humidité relative et 294 K.

4. Composition cosmétique selon la revendication 1, dans laquelle la matière particulaire est insoluble dans la composition à la fois avant et pendant l'emploi.

5. Composition cosmétique selon la revendication 1, comprenant en plus un (ou plusieurs) tensioactif choisi parmi les tensioactifs anioniques, non ioniques, amphotères et zwitterioniques et leurs mélanges.

6. Composition cosmétique selon la revendication 1, comprenant en plus un (ou plusieurs) agent épaississant ou de mise en suspension.

7. Procédé cosmétique d'enlèvement des cellules mortes ou indésirables de la surface du cuir chevelu ou de la peau consistant à lui appliquer et à masser ou à travailler une composition exfoliante comportant une matière exfoliante particulaire avec une granulométrie dans la gamme des 0,03 à 3 mm, caractérisée en ce que la matière particulaire est friable et dans des conditions d'utilisation de la composition se rompt en particules ayant une dimension moyenne inférieure à 40 microns.

8. Utilisation d'une matière exfoliante dans une composition cosmétique d'une matière particulaire comprenant des particules qui sont friables dans des conditions d'utilisation de la composition.
